# EUROPÄISCHE PATENTSCHRIFT

(11) **EP 4 370 500 B1**
(45) Veröffentlichungstag und Bekanntmachung des Hinweises auf die Patenterteilung: **26.03.2025**
(21) Anmeldenummer: 22747334.5
(22) Anmeldetag: 08.07.2022
(51) Int. Cl.: C07C 267/00, C07C 269/02, C07C 271/28, C08K 5/29, C08G 18/02

(54) **AROMATISCHE CARBODIIMIDE, VERFAHREN ZU DEREN HERSTELLUNG UND DEREN VERWENDUNG**
NOVEL CARBODIIMIDES WITH UREA AND/OR URETHANE TERMINAL GROUPS, METHOD FOR THEIR PREPARATION AND THEIR USE
NOUVEAUX CARBODIIMIDES AYANT DES GROUPES FINAUX URÉE ET/OU URÉTHANE, LEUR PROCÉDÉ DE PRODUCTION ET LEUR UTILISATION

(30) Priorität: 14.07.2021 EP 21185586
(43) Veröffentlichungstag der Anmeldung: 22.05.2024
(73) Patentinhaber: LANXESS Deutschland GmbH, 50569 Köln (DE)
(72) Erfinder: LAUFER, Wilhelm, 67158 Ellerstadt (DE)
(86) Internationale Anmeldenummer: PCT/EP2022/069107
(87) Internationale Veröffentlichungsnummer: WO 2023/285310

(56) Entgegenhaltungen:
- WO-A1-2014/184116
- CN-A- 105 778 026

## Beschreibung

Carbodiimide haben sich in vielen Anwendungen bewährt, z.B. als Hydrolyseschutzmittel für Thermoplaste, Polyole, Polyurethane, Triglyceride und Schmierstofföle.

Bevorzugt werden hierfür stark sterisch gehinderte Polycarbodiimide eingesetzt, die allerdings aus sehr speziellen Rohstoffen hergestellt werden und damit sehr teuer in der Anschaffung sind. Stark sterisch gehinderte Carbodiimide, wie z.B. solche auf Basis Triisopropylphenylisocyanat, haben darüber hinaus sehr hohe Schmelzpunkte, sind nicht löslich und können nicht oder nur mit einem beträchtlichen apparativen und zeitlichen Aufwand in die Ausgangsstoffe der Polyurethane eingebracht werden. Aromatische Carbodiimide, die auf preiswerteren Rohstoffen basieren, wie z.B. in EP 2997010 B1 beschrieben, können zwar in manchen Ester-basierten Polymeren wie PET oder PLA eine sehr gute Hydrolyseschutzwirkung erzielen, haben jedoch den Nachteil, dass sie in anderen Anwendungen wie z. B. in Polyurethan nicht ausreichend gegen Hydrolyse schützen und können damit nicht universell eingesetzt werden. Oftmals liegen die Carbodiimide des Stands der Technik in einer schlecht zu dosierenden Form, insbesondere in Form einer klebrigen Masse vor. CN105778026A offenbart Carbodiimide, ihre Herstellung und ihre Anwendung als Hydrolysestabilisatoren in Polyurethanen, wobei jedoch Nachteile bei der Löslichkeit in Polyester und der Dosierbarkeit inhärent sind.

Es besteht daher ein Bedarf an neuen Carbodiimiden, welche die Nachteile des Stands der Technik nicht aufweisen, einfach herzustellen sind, über eine hohe thermische Stabilität verfügen, auch in Polyurethan-Anwendungen einen exzellenten Hydrolyseschutz erzielen und zudem leichter zu dosieren sind.

Überraschenderweise konnte diese Aufgabe gelöst werden durch Carbodiimide der Formel (I) 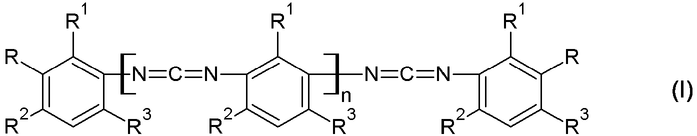 in der
R gleich oder verschieden sein kann und ausgewählt ist aus -NCN-R^{I}, und -NHCOOR^{III}, wobei
R^{I} C₁-C₂₂-Alkyl, C₆-C₁₂-Cycloalkyl, C₆-C₁₈-Aryl oder C₆-C₁₈-Aralkyl, bevorzugt Triisopropylphenyl bedeutet und
R^{III} für einen alkylierten Polyoxyalkylenrest steht,
R¹, R² und R³ jeweils unabhängig voneinander für Methyl, i-Propyl- oder n-Propyl steht, wobei an jedem Benzolring einer der Reste R¹, R² und R³ Methyl ist und n von 0 bis 500, bevorzugt n von 1 bis 50 ist.

Die Molmasse des alkylierten Polyoxyalkylenrests beträgt bevorzugt mindestens 200 g/Mol, besonders bevorzugt von 200 - 600 g/mol und meist bevorzugt von 350 - 550 g/mol.

Der Carbodiimid-Gehalt (NCN-Gehalt, gemessen durch Titration mit Oxalsäure) der erfindungsgemäßen Carbodiimide liegt üblicherweise bei 2 - 17 Gew.%. Zur Bestimmung des NCN-Gehaltes werden die NCN-Gruppen mit im Überschuss zugegebener Oxalsäure reagiert und dann die nicht reagierte Oxalsäure mit Natriummethylat potentiometrisch rücktitriert, wobei der Blindwert des Systems berücksichtigt wird.

Bevorzugt sind Carbodiimide, wobei R¹, R² und R³ jeweils unabhängig voneinander für Methyl- oder i-Propyl- stehen.

Bevorzugt sind Carbodiimide der Formel (I) worin R für NCN-R^{I} steht, wobei n von 0 bis 500, bevorzugt von 1 bis 100 und meist bevorzugt von 1 bis 50 ist und der Carbodiimid-Gehalt vorzugsweise 10 - 17 Gew.%, besonders bevorzugt 11 - 15 Gew.% und meist bevorzugt 13 - 14 Gew.% beträgt.

Eine weitere bevorzugte Ausführungsform betrifft Carbodiimide der Formel (I) worin R für NHCOOR^{III} steht, wobei n von 0 bis 20, bevorzugt von 1 bis 10, besonders bevorzugt von 3 bis 8 ist und der Carbodiimid-Gehalt vorzugsweise von 4 bis 13 Gew.%, besonders bevorzugt von 10 bis 13 Gew.% beträgt.

Carbodiimide der Formel (I) mit R = -NCN-R^{I}, wobei R^{I} die obige Bedeutung hat und R¹, R² und R³jeweils unabhängig für Methyl- oder i-Propyl- steht, wobei an jedem Benzolring einer der Reste R¹, R² und R³ Methyl ist, sind fest und weisen Erweichungspunkte > 40°C auf. Sie eignen sich daher hervorragend für die Stabilisierung von esterbasierten Polymeren, vorzugsweise Polymeren ausgewählt aus Polyesterpolyolen, Polyethylenterephthalat (PET), Polybutylenterephthalat (PBT), Polytrimethylenterephthalat (PTT), Copolyestern wie modifizierten Polyestern aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends.

Die Erfindung betrifft auch ein Verfahren zur Stabilisierung der esterbasierten Polymere durch Zugabe der vorgenannten Carbodiimide. Bevorzugt werden die vorgenannten Carbodiimide zu den esterbasierten Polymeren dabei mittels Feststoffdosieraggregaten zugegeben.

Feststoffdosieraggregate im Sinne der Erfindung sind vorzugsweise: Ein-, Zwei- und Mehrwellenextruder, kontinuierlich arbeitenden Co-Kneter (Typ Buss) und diskontinuierlich arbeitenden Kneter, z.B. vom Typ Banbury.

In einer weiteren Ausführung der Erfindung sind Carbodiimide der Formel (I) bevorzugt, wobei R für -NHCOOR^{III} und R^{III} für alkyliertes Polyoxyalkylen und R¹, R² und R³ jeweils unabhängig voneinander für Methyl- oder i-Propyl- stehen, n von 0 bis 20, bevorzugt n von 1 bis 10, besonders bevorzugt von 1 bis 4 und meist bevorzugt von 2 bis 3 ist und der Carbodiimid-Gehalt vorzugsweise von 2 bis 10 Gew.%, besonders bevorzugt bei 4 bis 8 Gew.% und meist bevorzugt von 5 bis 7 Gew.% beträgt.

Bevorzugte alkylierte Polyoxyalkylenreste sind monoalkylierte Polyethylenglykolether, besonders bevorzugt Polyethylenglykolmonomethylether, insbesondere solche mit Molmassen von 200 - 600 g/mol, bevorzugt von 350 - 550 g/mol.

Die vorgenannten Carbodiimide der Formel (I) mit R = -NHCOOR^{III}, wobei R^{III} ein alkylierter Polyoxyalkylenrest ist, sind üblicherweise bei Raumtemperatur flüssig und erlaubt daher - im Unterschied zu den meisten festen Carbodiimiden - zusätzlich die Einarbeitung in flüssige Polyesterpolyole, die für die Herstellung von TPU und PU-Schäume verwendet werden.

Die Erfindung betrifft daher auch ein Verfahren zur Stabilisierung von TPU und PU-Schäumen, wobei die vorgenannten Carbodiimide zu den flüssigen Polyesterpolyolen gegeben werden, aus denen die TPU und PU-Schäume erzeugt werden.

Die Erfindung betrifft weiterhin ein Verfahren zur Stabilisierung von Ester-basierten Ölen und/oder Schmierstoffen oder -fetten, wobei die vorgenannten Carbodiimide zu den esterbasierten Polymeren zugegeben werden.

Die Konzentration der erfindungsgemäßen Carbodiimide der Formel (I) in esterbasierten Polymeren, bzw. in Ester-basierten Ölen, Schmierstoffen oder -fetten beträgt vorzugsweise von 0,1 bis 5 Gew.%, bevorzugt von 0,5 bis 3 Gew.%, besonders bevorzugt von 1 bis 2 Gew.%.

Die erfindungsgemäßen Carbodiimide weisen vorzugsweise mittlere Molmassen (Mw) von 1000 bis 20000 g/mol, bevorzugt von 1500 bis 5000 g/mol, besonders bevorzugt von 2000 bis 4000 g/mol auf.

Weiterhin sind Carbodiimide bevorzugt, die eine Polydispersität D = Mw/Mn von 1,2 bis 2, besonders bevorzugt von 1,4 bis 1,8 aufweisen.

Der Rahmen der Erfindung erfasst alle oben stehenden und im Folgenden aufgeführten allgemeinen oder in Vorzugsbereichen genannten Restedefinitionen, Indizes, Parameter und Erläuterungen untereinander, also auch zwischen den jeweiligen Bereichen und Vorzugsbereichen in beliebiger Kombination.

Gegenstand der vorliegenden Erfindung ist zudem die Herstellung der erfindungsgemäßen Carbodiimide durch Carbodiimidisierung von aromatischen Diisocyanaten der Formel (II) 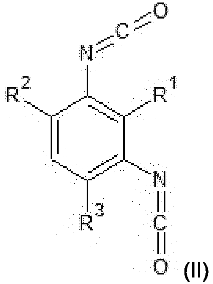 unter Abspaltung von Kohlendioxid bei Temperaturen von 80 °C bis 200 °C in Gegenwart von Katalysatoren und gegebenenfalls Lösungsmittel und der anschließenden Funktionalisierung der freien NCO-Gruppen mit Alkoholen der Formel NOR^{III}, wobei R¹ bis R³ und R^{I} bis R^{III} die für die Verbindungen der Formel (I) genannten Bedeutungen haben.

Bevorzugt werden in diesem Verfahren als aromatische Diisocyanate der Formel (II) aromatische Diisocyanate der Formeln (III) und/oder (IV) eingesetzt:

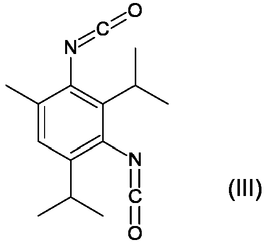

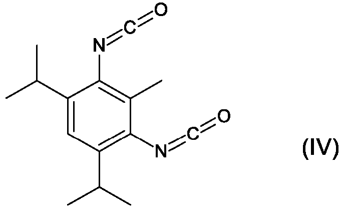

Besonders bevorzugt werden Mischungen der Diisocyanate der Formeln (III) und (IV) eingesetzt, vorzugsweise im Verhältnis 60 : 40 bis 95 : 5, besonders bevorzugt 70 : 30 bis 90 : 10.

Die für die Herstellung der Diisocyanate notwendigen aromatischen Diamine können - wie dem Fachmann bekannt- über eine Friedel-Crafts-Alkylierung der entsprechenden Toluoldiamine mit dem entsprechenden Alken, oder Halogenalkan hergestellt werden. Anschließend werden diese Diamine mit Phosgen zum entsprechenden Diisocyanat umgesetzt.

Zur Herstellung der erfindungsgemäßen Carbodiimide können die Diisocyanate z. B. der Formel (III) und/oder (IV) bei erhöhter Temperaturen, vorzugsweise Temperaturen von 80 - 200 °C, besonders bevorzugt von 100 bis 180°C, ganz besonders bevorzugt von 120 - 140°C, zweckmäßigerweise in Gegenwart von Katalysatoren und ggf. eines weiteren Monoisocyanat der Gruppe R^{I} unter Kohlendioxidabspaltung kondensiert werden. Hierfür geeignete Verfahren werden beispielsweise beschrieben in DE-A 1130594 und DE-A 11564021.

Als Katalysatoren für die Herstellung der Verbindungen der Formel (I) sind in einer Ausführungsform der Erfindung Phosphorverbindungen bevorzugt. Als Phosphorverbindungen werden vorzugsweise Phospholenoxide, Phospholidine oder Phospholinoxide sowie die entsprechenden Phospholensulfide verwendet. Ferner können als Katalysatoren tertiäre Amine, basisch reagierende Metallverbindungen, Alkali-, Erdalkalioxide oder -Hydroxide, -Alkoholate oder -Phenolate, Carbonsäuremetallsalze und nicht basische Organometallverbindungen verwendet werden.

Die Carbodiimidisierung kann sowohl in Substanz als auch in einem Lösemittel durchgeführt werden. Als Lösemittel werden vorzugsweise Alkylbenzole, Paraffinöle, Polyethylenglykoldimethylether, Ketone oder Lactone eingesetzt.

In einer Ausführungsform der vorliegenden Erfindung wird hierzu die Temperatur der Reaktionsmischung auf 50 - 120 °C, bevorzugt 60 - 100 °C, besonders bevorzugt auf 80 - 90 °C reduziert und die Katalysatoren werden unter verminderten Druck abdestilliert. In einer bevorzugten Herstellungsvariante der erfindungsgemäßen Carbodiimide wird anschließend das überschüssige Diisocyanat bei Temperaturen von 150 - 200°C, bevorzugt 160 - 180°C abdestilliert. Anschließend werden die freien endständigen Isocyanatgruppen der Carbodiimide mit Alkoholen vorzugsweise in einem geringem Überschuss an -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinndilaurat oder DOTL (Dioctylzinndilaurat), abreagiert. Das Stoffmengen-Verhältnis (molares Verhältnis) von Alkoholen zu Carbodiimiden liegt vorzugsweise bei 1,005-1,05 : 1, besonders bevorzugt bei 1,01-1,03 : 1, bezogen auf die vorhandenen N=C=O-Gruppen.

In einer weiteren Ausführungsform der vorliegenden Erfindung wird zur Unterbrechung der Carbodiimidisierung die Temperatur der Reaktionsmischung auf einen Wert im Bereich von 50 bis 120 °C, bevorzugt von 60 bis 100 °C, besonders bevorzugt auf von 80 bis 90 °C reduziert und gegebenenfalls nach Zugabe eines Lösemittels, bevorzugt ausgewählt aus der Gruppe der Alkylbenzole, besonders bevorzugt Toluol, werden die freien endständigen Isocyanatgruppen der Carbodiimide mit Alkoholen vorzugsweise in einem geringem Überschuss an -OH-Gruppen, gegebenenfalls in Anwesenheit eines dem Fachmann bekannten PU-Katalysators, vorzugsweise tert. Aminen oder Organozinn-Verbindungen, besonders bevorzugt DBTL (Dibutylzinn dilaurat oder DOTL (Dioctylzinndilaurat), abreagiert. Das Stoffmengen-Verhältnis von Alkoholen zu Carbodiimiden liegt vorzugsweise bei von 1,005 bis 1,05 : 1, besonders bevorzugt bei von 1,01 bis 1,03 : 1, bezogen auf die vorhandenen N=C=O-Gruppen.

Nach vollständiger Reaktion wird der Katalysator und gegebenenfalls das Lösemittel vorzugsweise bei Temperaturen von 80-200°C unter verminderten Druck abdestilliert.

Gegenstand der vorliegenden Erfindung ist zudem ein weiteres Verfahren zur Herstellung der erfindungsgemäßen Carbodiimide durch eine partielle, vorzugsweise < 50% ige Funktionalisierung der freien NCO-Gruppen von aromatischen Diisocyanaten der Formel (II) 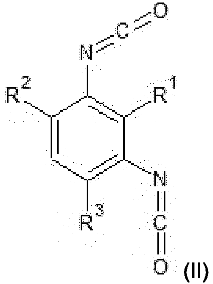 mit Alkoholen der Formel HOR^{III} und anschließende Carbodiimidisierung unter Abspaltung von Kohlendioxid bei Temperaturen von 80 °C bis 200 °C in Gegenwart von Katalysatoren und gegebenenfalls Lösungsmittel, wobei R¹ bis R³ und R^{III} die für die Verbindungen der Formel (I) genannten Bedeutungen haben.

Bevorzugt werden auch in diesem Verfahren als aromatische Carbodiimide der Formel (II) aromatische Carbodiimide der Formel (III) 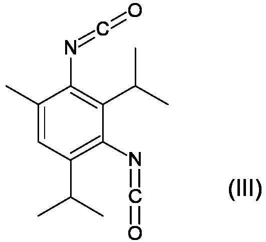 und/oder der Formel (IV) 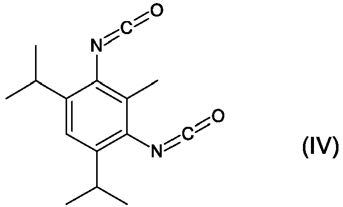 eingesetzt.

Vorzugsweise werden die erfindungsgemäßen Carbodiimide nach ihrer Herstellung gereinigt. Die Reinigung der Rohprodukte kann destillativ und/oder mittels Extraktion mit Lösemitteln erfolgen. Als geeignete Lösemittel für die Aufreinigung können vorzugsweise Polyethylenglykoldimethylether, Alkylbenzole, Paraffinöle, Alkohle, Ketone oder Ester eingesetzt werden. Dabei handelt es sich um handelsübliche Lösemittel.

Gegenstand der vorliegenden Erfindung ist zudem die Herstellung der erfindungsgemäßen Carbodiimide durch Carbodiimidisierung von aromatischen Diisocyanaten der Formel (II) 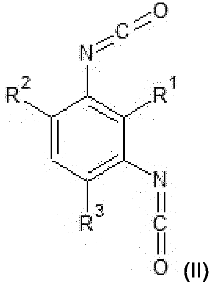 unter Abspaltung von Kohlendioxid bei Temperaturen von 80 °C bis 200 °C in Gegenwart von Katalysatoren und gegebenenfalls Lösungsmittel, wobei vor, während oder nach der Carbodiimidisierung der Diisocyanate Monoisocyanate der Formel OCN-R^{I} zugesetzt werden und wobei R¹ bis R³ und R^{I} die für die Verbindungen der Formel (I) genannten Bedeutungen haben. Im Falle der Zugabe nach Carbodiimidisierung der Diisocyanate wird die Carbodiimidisierung weitergeführt um verbleibende Isocyanat(end)Gruppen von Carbodiimiden mit Monoisocyanat in Gruppen der Formel -NCN-R^{I} zu überführen. Im Falle der Zugabe der Monoisocyanate vor oder während der Carbodiimidisierung der Diisocyanate erfolgt die Funktionalisierung der Endgruppen von selbst während der Carbodiimidisierung.

Bevorzugt werden auch in diesem Verfahren als aromatische Diisocyanate der Formel (II) aromatische Diisocyanate der Formel (III) 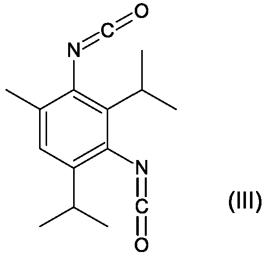 und/oder der Formel (IV) 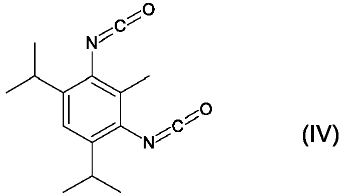 eingesetzt.

Als Monoisocyanat wird bevorzugt Triisopropylphenylisocyanat eingesetzt.

Gegenstand der vorliegenden Erfindung ist weiterhin eine Zusammensetzung enthaltend
- mindestens ein esterbasiertes Polymer, und
- mindestens ein erfindungsgemäßes Carbodiimid der Formel (I).

Bei den esterbasierten Polymeren handelt es sich vorzugsweise um Polymere ausgewählt aus Polyesterpolyolen, Polyethylenterephthalat (PET), Polybutylenterephthalat (PBT), Polytrimethylenterephthalat (PTT), Copolyestern wie modifizierten Polyestern aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends.

In einer besonders bevorzugten Ausführungsform der Erfindung handelt es sich bei den Estergruppen-enthaltenden Polymeren um thermoplastischen Polyurethane (TPU).

Dabei beträgt die Konzentration des erfindungsgemäßen Carbodiimides der Formel (I) in der erfindungsgemäßen Zusammensetzung vorzugsweise 0,1 - 5 Gew.%, bevorzugt 0,5 - 3 Gew.%, besonders bevorzugt 1 - 2 Gew.%.

Bei den Polyesterpolyolen als esterbasierte Polymere handelt es sich vorzugsweise um langkettige Verbindungen, die vorzugsweise ein Molekulargewicht (in g/mol) von bis zu 2000, bevorzugt zwischen 500 - 2000 und besonders bevorzugt zwischen 500 - 1000, aufweisen.

Der Begriff Polyesterpolyole im Sinne der Erfindung umfasst dabei sowohl langkettige Diole als auch Triole, wie auch Verbindungen mit mehr als drei Hydroxylgruppen je Molekül.

Vorteilhaft ist es, wenn das Polyesterpolyol eine OH-Zahl von bis zu 200, vorzugsweise zwischen 20 und 150 und besonders bevorzugt zwischen 50 und 115, aufweist. Insbesondere eignen sich Polyesterpolyole, die Reaktionsprodukte von verschiedenen Polyolen mit aromatischen oder aliphatischen Dicarbonsäuren und/oder Polymere von Lactonen sind.

Bei den im Sinne der Erfindung eingesetzten Polyesterpolyolen handelt es sich um handelsübliche Verbindungen, die bei der Firma Covestro Deutschland AG unter dem Handelsnamen Baycoll^{®} oder Desmophen^{®} erhältlich sind.

Ein weiterer Gegenstand der vorliegenden Erfindung ist ein Verfahren zur Herstellung der erfindungsgemäßen Carbodiimide der Formel (I) mit R = -NCN-R^{I}, bei dem die Schmelze nach der Carbodiimidisierung ungereinigt oder nach Aufreinigung pastilliert wird, vorzugsweise auf Pastillierbändern. Dabei sind gängige Pastilliersysteme ebenso wie gängige Granuliersysteme einsetzbar. Diese sind z.B. erhältlich bei der Firma Sandvik Holding GmbH oder der Firma GMF Gouda.

Ganz besonders geeignet sind die erfindungsgemäßen Carbodiimide der Formel (I) mit R = -NCN-R^{I} , und R^{I} = Triisopropylphenyl.

Gegenstand der vorliegenden Erfindung ist zudem die Verwendung der erfindungsgemäßen Carbodiimide als Schutz gegen hydrolytischen Abbau in esterbasierten Polymeren, vorzugsweise Polymeren ausgewählt aus Polyesterpolyolen, Polyethylenterephthalat (PET), Polybutylenterephthalat (PBT), Polytrimethylenterephthalat (PTT), Copolyestern wie modifizierten Polyestern aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends, oder in Triglyceriden, vorzugsweise Trimethylolpropantrioleat (TMP-Oleat), in Ölformulierungen für die Schmierstoffindustrie, in PU-Klebstoffen, in PU-Gießharzen. Die Verwendung in Urethanen umfasst dabei u.a. PU-Schäume, PU-Beschichtungen für Holz, Leder, Kunstleder und Textilien. Besonders bevorzugt ist eine Verwendung in thermoplastischen Polyurethanen (TPU).

Die nachfolgenden Beispiele dienen der Erläuterung der Erfindung, ohne dabei limitierend zu wirken.

### Ausführungsbeispiele

### Getestet wurden:

1) CDI A: ein festes Carbodiimid mit einem NCN-Gehalt von ca. 12 Gew.%, auf Basis von ca. 80 Gew.% 2,4-Diethyltoluoyldiisocyanat und 20 Gew.% 2,6-Diethyltoluoyldiisocyanat endfunktionalisiert mit Cyclohexanol (Vergleichsbeispiel analog EP 2997010 B1).
2) CDI (B): ein hochviskoses Carbodiimid mit einem NCN-Gehalt von ca. 14 Gew.%, auf Basis von 80 Gew.% 2,4-Diethyltoluoyldiisocyanat und 20 Gew.% 2,6-Diethyltoluoyldiisocyanat endfunktionalisiert mit -NCN-R^{I}, wobei R^{I} = Triisopropylphenyl und n > 40 (Vergleichsbeispiel).
3) CDI (C): ein festes polymeres Carbodiimid mit einem NCN-Gehalt von ca. 14 Gew.% auf Basis von Diisopropyltoluoyldiisocyanat (Formel III und IV im Gewicht-Verhältnis von ca. 1:4), endfunktionalisiert mit -NCN-R^{I}, wobei R^{I} = Triisopropylphenyl und n > 40 (erfindungsgemäßes Beispiel).
4) CDI (D): ein festes polymeres Carbodiimid auf Basis von Diisopropyltoluoyldiisocyanat, endfunktionalisiert mit Ethylamin (Vergleichsbeispiel analog CN 105778026).
5) CDI (E): ein festes polymeres Carbodiimid mit einem NCN-Gehalt von ca. 6 - 7 Gew.% auf Basis von Diisopropyltoluoyldiisocyanat (Formel III und IV im Gewicht-Verhältnis von ca. 1:4), endfunktionalisiert mit Methylpoylethylenglykol (Mw ca. 550 g/mol), wobei n = 4 - 5 (erfindungsgemäßes Beispiel).

### Ester-basierte Polymere:

6) Nicht stabilisiertes thermoplastisches Polyurethan (TPU) erhältlich bei der Covestro AG unter den Namen Desmopan.

### Herstellung der Carbodiimide CDI (A)

In einem ausgeheizten und mit Stickstoff befüllten 250 ml Vierhalskolben wurden unter Stickstoffstrom 150 g Diisocyanate und 37,5g Cyclohexanol vorgelegt. Nach Zugabe von 50 mg 1-Methyl-phospholenoxid wurde langsam auf 180 °C erwärmt. Im Anschluss wurde bei 180 °C solange carbodiimidisiert, bis ein NCO-Gehalt von < 1 Gew.% erreicht worden war.

### Herstellung des Carbodiimides CDI (B) und CDI (C):

In einem ausgeheizten und mit Stickstoff befüllten 250 ml Vierhalskolben wurden unter Stickstoffstrom 150 g die Diisocyanate und 37,5g Triisopropylphenylisocyanat vorgelegt. Nach Zugabe von 50 mg 1-Methyl-phospholenoxid wurde langsam auf 180 °C erwärmt. Im Anschluss wurde bei 180 °C solange carbodiimidisiert, bis ein NCO-Gehalt von < 1 Gew.% erreicht worden war.

### Herstellung des Carbodiimides CDI (D)

In einem ausgeheizten und mit Stickstoff befüllten 250 ml Vierhalskolben wurden unter Stickstoffstrom 150 g die Diisocyanate und 7,0 g Ethylamin vorgelegt. Nach Zugabe von 50 mg 1-Methyl-phospholenoxid wurde langsam auf 180 °C erwärmt. Im Anschluss wurde bei 180 °C solange carbodiimidisiert, bis ein NCO-Gehalt von < 0,1 Gew.% erreicht worden war.

### Herstellung des Carbodiimides CDI (E)

In einem ausgeheizten und mit Stickstoff befüllten 250 ml Vierhalskolben wurden unter Stickstoffstrom 150 g die Diisocyanate und 100 g MPEG (Methylpolyethylenglycol, Mw von ca. 550 g/mol) vorgelegt. Nach Zugabe von 50 mg 1-Methyl-phospholenoxid wurde langsam auf 180 °C erwärmt. Im Anschluss wurde bei 180 °C solange carbodiimidisiert, bis ein NCO-Gehalt von < 0,1 Gew.% erreicht worden war.

### Hydrolyseschutz in thermplastischem Polyurethan (TPU)

Zur Bewertung der Hydrolyseschutzwirkung in TPU wurden je 1,5 Gew.% der untersuchten Carbodiimide mittels eines Labordoppelschneckenextruders ZSK 25 der Firma Werner & Pfleiderer vor der unten beschriebenen Messung in TPU eindispergiert. Aus den gewonnenen Granulaten wurden dann die für die Messung der Reißfestigkeit verwendeten Normprüfkörper an einer Spritzgießmaschine des Typs Arburg Allrounder 320 S 150-500 hergestellt.

Für den Hydrolysetest wurden diese Normprüfkörper in Wasser bei einer Temperatur von 80°C gelagert und deren Reißfestigkeit in MPa gemessen.

Die Ergebnisse sind in der Tabelle 1 aufgelistet:

**Tabelle 1**

| **Reißfestigkeit (MPa)** | **Bsp. 1 (vgl.) (TPU)** | **Bsp. 2 (vgl.) (TPU, CDI A)** | **Bsp. 3 (vgl) (TPU/CDI D)** | **Bsp. 4 (erf.) (TPU/CDI E)** |
|---|---|---|---|---|
| 0 Tage | 30 | 30 | 31 | 32 |
| 5 Tage | 28 | 30 | 30 | 32 |
| 10 Tage | 26 | 30 | 30 | 31 |
| 15 Tage | 12 | 28 | 30 | 30 |
| 20 Tage | 6 | 25 | 30 | 30 |
| 30 Tage | 0 | 5 | 28 | 30 |
| 80 Tage | - | - | 18 | 30 |
| 85 Tage | - | - | 5 | 29 |
| 90 Tage | - | - | | 28 |

| | | | | |
|---|---|---|---|---|
| vgl. =Vergleichsbeispiel, erf.= erfindungsgemäß | | | | |

Die Ergebnisse aus der Tabelle 1 zeigen, dass die erfindungsgemäßen Carbodiimide im Vergleich zum Stand der Technik einen deutlich besseren Hydrolyseschutz in TPU bewirken.

### Löslichkeit in Polyesterpolyol

Grundsätzlich erfolgt die Stabilisierung der Ester-basiertem TPU-Elastomere gegen Hydrolyse direkt bei der Herstellung. Dazu wird standardmäßig das Carbodiimid zu dem Polyesterpolyol oder Polyester-Weichmacher zugegeben bevor die Reaktion mit Isocyatanen zum Polyurethan erfolgt. Aus diesem Grund spielt die Löslichkeit des Carbodiimides eine wichtige Rolle. In Tabelle 2 werden die Verschiedene Carbodiimide in einem Standard-Polyesterpolyol basierend auf Adipinsäure und Ethandiol mit einem Mw = 2000 (Desmophen 2000 MM der Firma Coverstro AG) bei 80 °C dargestellt.

**Tabelle 2**

| | | |
|---|---|---|
| Löslichkeit in Polyesterpoylol | Bsp. 5 (vgl.) (CDl D) | Bsp. 6 (erf.) (CDI E) |
| | Nicht löslich | Löslich |

### Hydrolyseschutz in Polyethylenterephthalat (PET)

Zur Bewertung der Hydrolyseschutzwirkung in PET wurden je 1,5 Gew.% der untersuchten Carbodiimide mittels eines Labordoppelschneckenextruders ZSK 25 der Firma Werner & Pfleiderer vor der unten beschriebenen Messung in PET eindispergiert. Aus den gewonnenen Granulaten wurden dann die für die Messung der Reißfestigkeit verwendeten F3-Normprüfkörper an einer Spritzgießmaschine des Typs Arburg Allrounder 320 S 150 - 500 hergestellt.

Für den Hydrolysetest wurden diese F3-Normprüfkörper in Wasser bei einer Temperatur von 90°C gelagert und deren Reißfestigkeit in MPa gemessen. Aufgetragen sind in Tabelle 2 die relativen Reißfestigkeiten = (Reißfestigkeit nach x Tagen Lagerung / Reißfestigkeit nach 0 Tagen) x 100. Als untere Grenze für relative Reißfestigkeit gilt meist ein Wert von 70 - 75 %.

Die Ergebnisse sind in der Tabelle 3 aufgelistet:

**Tabelle 3**

| **Relative Reißfestigkeit (%)** | **Bsp. 7 (vgl.) (PET)** | **Bsp. 8 (vgl.) (PET/CDI A)** | **Bsp. 9 (erf.) (PET/CDI C)** |
|---|---|---|---|
| 0 Tage | 100 | 100 | 100 |
| 5 Tag | 100 | 100 | 100 |
| 10Tage | 51 | 100 | 100 |
| 15 Tage | - | 100 | 100 |
| 20 Tage | - | 100 | 100 |
| 25 Tage | - | 52 | 81 |
| 28 Tage | - | - | 41 |

| | | | |
|---|---|---|---|
| vgl. =Vergleichsbeispiel, erf.= erfindungsgemäß | | | |

### Versuche zur Pastillierbarkeit und Dosierbarkeit der festen Carbodiimide

Zur Klärung der Konfektionierbarkeit, Handhabung und Dosierbarkeit der verschiedenen festen Carbodiimide wurden diese bezüglich Aussehen, Pastillierbarkeit und Erweichungspunkt verglichen. Die Erweichungspunkte wurden mittels einer Koffler-Bank bestimmt.

Die Ergebnisse sind in der Tabelle 4 aufgelistet:

| Carbodiimid | Aussehen (bei RT) | Pastillierbarkeit | Dosierbarkeit (T bis 40 °C) | Erweichungspunkt (°C) |
|---|---|---|---|---|
| CDI (B), vgl. | weiche, klebrige Masse | nicht möglich | - | < 20 |
| CDI (D), vgl. | harte, klebrige Masse | nicht möglich | - | > 120 |
| CDI (C), erf. | fest, spröde | sehr gut | sehr gut | ca. 80 |

| | | | | |
|---|---|---|---|---|
| vgl. =Vergleichsbeispiel, erf.= erfindungsgemäß | | | | |

Die Ergebnisse der Tabelle 4 zeigen, dass die erfindungsgemäßen Carbodiimide auf Basis von Diisopropyltoluoyldiisocyanat endgekappt mit einem Monoisocyanat im Vergleich zum polymeren Carbodiimid auf Basis von Diethyltoluoyldiisocyanat ebenfalls endgekappt mit einem Monoisocyanat und im Vergleich zu Carbodiimid D eine hervorragende Pastillierbarkeit und einen hohen Erweichungspunkt aufweisen und damit Vorteile bei der Konfektionierung und Dosierung des Feststoffes bei der Stabilisierung der Ester-basierten Polymere mit sich bringen. Das erfindungsgemäße Carbodiimid E ist flüssig und kann als solche dosiert werden.

## Patentansprüche

1. Carbodiimide der Formel (I) 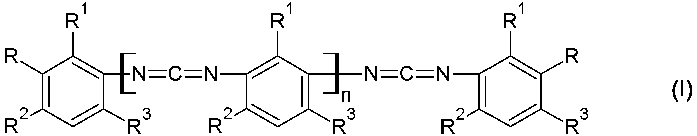 in der
R gleich oder verschieden sein kann und ausgewählt ist aus -NCN-R^{I} - und -NHCOOR^{III}, wobei
R^{I} C₁-C₂₂-Alkyl, C₆-C₁₂-Cycloalkyl, C₆-C₁₈-Aryl oder C₆-C₁₈-Aralkyl, bevorzugt Triisopropylphenyl bedeutet und
R^{III} für einen alkylierten Polyoxyalkylenrest steht,
R¹, R² und R³ jeweils unabhängig voneinander für Methyl, i-Propyl, oder n-Propyl steht, wobei an jedem Benzolring einer der Reste R¹, R² und R³ Methyl ist und n von 0 bis 500, bevorzugt von 1 bis 50 ist.

2. Carbodiimide gemäß Anspruch 1, wobei R¹, R² und R³ jeweils unabhängig voneinander für Methyl- oder i-Propyl- steht.

3. Carbodiimide gemäß Anspruch 1 oder 2, wobei der Carbodiimid-Gehalt 2 - 17 Gew.% beträgt

4. Carbodiimide gemäß einem oder mehreren der Ansprüche 1 bis 3, wobei in Formel (I) R für NCN-R^{I} steht n von 0 bis 500, bevorzugt von 1 bis 100, besonders bevorzugt von 1 bis 50 ist und der Carbodiimid-Gehalt vorzugsweise 10 - 17 Gew.%, besonders bevorzugt 11 - 15 Gew.% und meist bevorzugt 13 - 14 Gew.% beträgt.

5. Carbodiimide gemäß einem oder mehreren der Ansprüche 1 bis 3, wobei in Formel (I) R für -NHCOOR^{III} steht, n von 0 bis 20, bevorzugt von 1 bis 10, besonders bevorzugt von 3 bis 8 und der Carbodiimid-Gehalt vorzugsweise von 4 bis 13 Gew.%, besonders bevorzugt von 10 bis 13 Gew.% beträgt.

6. Carbodiimide gemäß einem oder mehreren der Ansprüche 1 bis 3, wobei in Formel (I) R für -NHCOOR^{III} und R^{III} für einen alkylierten Polyoxyalkylenrest steht, n von 0 bis 20, bevorzugt n von 1 bis 10, besonders bevorzugt von 1 bis 4 und meist bevorzugt von 2 bis 3 ist und der Carbodiimid-Gehalt vorzugsweise von 2 - 10 Gew.%, besonders bevorzugt bei 4 - 8 Gew.% und meist bevorzugt von 5 - 7 Gew.% beträgt.

7. Carbodiimide gemäß Anspruch 6, wobei R^{III} für monoalkylierte Polyethylenglykolether, bevorzugt Polyethylenglykolmonomethylether steht, vorzugsweise mit Molmassen von 200 - 600 g/mol, besonders bevorzugt mit Molmassen von 350 - 550 g/mol.

8. Verfahren zur Herstellung von Carbodiimiden gemäß einer oder mehrerer der Ansprüche 1 bis 7 umfassend die Schritte:
a) Carbodiimidisierung von aromatischen Diisocyanaten der Formel (II) 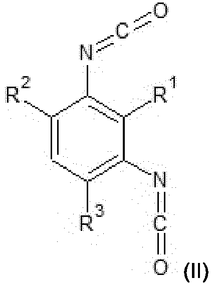 unter Abspaltung von Kohlendioxid bei Temperaturen von 80 °C bis 200 °C in Gegenwart von Katalysatoren und gegebenenfalls Lösungsmittel und
b) Funktionalisierung der freien NCO-Gruppen der in Schritt a) erhaltenen Carbodiimide mit Alkoholen der Formel NOR^{III}, wobei R¹ bis R³ und R^{III} die für die Verbindungen der Formel (I) genannten Bedeutungen haben.

9. Verfahren zur Herstellung von Carbodiimiden gemäße einem oder mehreren der Ansprüche 1 bis 7 umfassend die Schritte:
a) partielle, vorzugsweise < 50%ige Funktionalisierung der freien NCO-Gruppen von aromatischen Diisocyanaten der Formel (II) 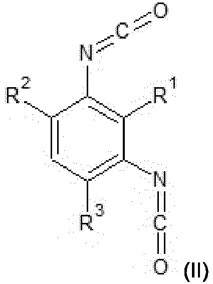 mit Alkoholen der Formel NOR^{III} und
b) anschließende Carbodiimidisierung der in Schritt a) erhaltenen partiell funktionalisierten, aromatischen Diisocyanate der Formel (II) unter Abspaltung von Kohlendioxid bei Temperaturen von 80 °C bis 200 °C in Gegenwart von Katalysatoren und gegebenenfalls Lösungsmittel,
wobei R¹ bis R³ und R^{III} die für die Verbindungen der Formel (I) genannten Bedeutungen haben

10. Verfahren zur Herstellung von Carbodiimiden gemäß einer oder mehrerer der Ansprüche 1 bis 7 umfassen die Carbodiimidisierung von aromatischen Diisocyanaten der Formel (II) 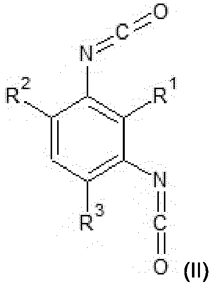 unter Abspaltung von Kohlendioxid bei Temperaturen von 80 °C bis 200 °C in Gegenwart von Katalysatoren und gegebenenfalls Lösungsmittel, wobei vor, während oder nach der Carbodiimidisierung der Diisocyanate Monoisocyanate der Formel OCN-R^{I} zugesetzt werden und wobei R¹ bis R³ und R^{I} die für die Verbindungen der Formel (I) genannten Bedeutungen haben.

11. Verfahren gemäß einem der Ansprüche 8 bis 10, wobei als aromatische Diisocyanate der Formel (II) Verbindungen der Formeln (III) 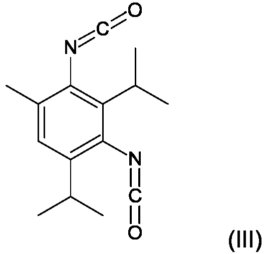 und/oder (IV) 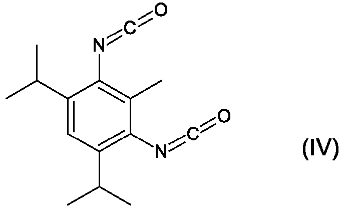 eingesetzt werden.

12. Verfahren gemäß einem oder mehreren der Ansprüche 8 bis 11, wobei R = -NCN-R¹, wobei R^{I} die für die Verbindungen der Formel (I) genannten Bedeutung hat und die bei der Carbodiimidisierung erhaltene Schmelze des Carbodiimids ungereinigt oder nach Aufreinigung pastilliert wird.

13. Verwendung der Carbodiimide nach einem der Ansprüche 1 bis 7 als Schutz gegen hydrolytischen Abbau in esterbasierten Polymeren, vorzugsweise Polymeren ausgewählt aus Polyesterpolyolen, Polyethylenterephthalat (PET), Polybutylenterephthalat (PBT), Polytrimethylenterephthalat (PTT), Copolyestern wie modifizierte Polyester aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren vorzugsweise thermoplastischen Polyurethanen (TPU), und Blends, wie vorzugsweise PA/PET- oder PHA/PLA-Blends.

14. Verwendung der Carbodiimide nach einem der Ansprüche 1 bis 8 als Schutz gegen hydrolytischen Abbau in thermoplastischem Polyurethan (TPU).

15. Zusammensetzung enthaltend mindestens ein erfindungsgemäßes Carbodiimid gemäß einem der Ansprüche 1 bis 7 und mindestens ein esterbasiertes Polymer, vorzugsweise ein esterbasiertes Polymer ausgewählt aus Polyesterpolyolen, Polyethylenterephthalat (PET), Polybutylenterephthalat (PBT), Polytrimethylenterephthalat (PTT), Copolyestern wie modifizierte Polyester aus Cyclohexandiol und Terephthalsäure (PCTA), thermoplastischen Polyester-Elastomeren (TPE E), Ethylenvinylacetat (EVA), Polymilchsäure (PLA) und/oder PLA-Derivaten, Polybutylenadipat-Terephthalaten (PBAT), Polybutylensuccinaten (PBS), Polyhydroxyalkanoaten (PHA), Polyurethan-Elastomeren insbesondere thermoplastischen Polyurethanen (TPU), und Blends, wie insbesondere PA/PET- oder PHA/PLA-Blends.

## Claims

1. Carbodiimides of formula (I) 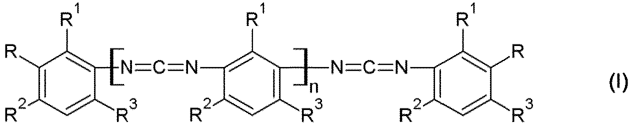 in which
R may be identical or different and is selected from -NCN-R^{I} and -NHCOOR^{III}, wherein
R^{I} represents C₁-C₂₂-alkyl, C₆-C₁₂-cycloalkyl, C₆-C₁₈-aryl or C₆-C₁₈-aralkyl, preferably triisopropylphenyl, and
R^{III} represents an alkylated polyoxyalkylene radical,
R¹, R² and R³ each independently of one another represent methyl, i-propyl or n-propyl, wherein on each benzene ring one of the radicals R¹, R² and R³ is methyl and n is from 0 to 500, preferably from 1 to 50.

2. Carbodiimides according to Claim 1, wherein R¹, R² and R³ each independently of one another represent methyl- or i-propyl-.

3. Carbodiimides according to Claim 1 or 2, wherein the carbodiimide content is 2-17% by weight.

4. Carbodiimides according to one or more of Claims 1 to 3, wherein in formula (I) R represents NCN-R^{I}, n is from 0 to 500, preferably from 1 to 100, particularly preferably from 1 to 50 and the carbodiimide content is preferably 10-17% by weight, particularly preferably 11-15% by weight and most preferably 13-14% by weight.

5. Carbodiimides according to one or more of Claims 1 to 3, wherein in formula (I) R represents -NHCOOR^{III}, n is from 0 to 20, preferably from 1 to 10, particularly preferably from 3 to 8, and the carbodiimide content is preferably from 4% to 13% by weight, particularly preferably from 10% to 13% by weight.

6. Carbodiimides according to one or more of Claims 1 to 3, wherein in formula (I) R represents -NHCOOR^{III} and R^{III} represents an alkylated polyoxyalkylene radical, n is from 0 to 20, preferably n is from 1 to 10, particularly preferably from 1 to 4 and most preferably from 2 to 3 and the carbodiimide content is preferably 2-10% by weight, particularly preferably 4-8% by weight and most preferably 5-7% by weight.

7. Carbodiimides according to Claim 6, wherein R^{III} represents monoalkylated polyethylene glycol ethers, preferably polyethylene glycol monomethyl ethers, preferably having molar masses of 200-600 g/mol, particularly preferably having molar masses of 350-550 g/mol.

8. Process for producing carbodiimides according to one or more of Claims 1 to 7 comprising the steps of:
a) carbodiimidization of aromatic diisocyanates of formula (II) 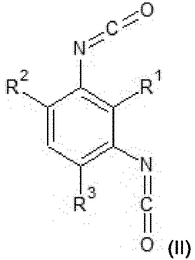 to eliminate carbon dioxide at temperatures of 80°C to 200°C in the presence of catalysts and optionally solvent and
b) functionalization of the free NCO groups of the carbodiimides obtained in step (a) with alcohols of formula NOR^{III}, wherein R¹ to R³ and R^{III} are as defined above for the compounds of formula (I).

9. Process for producing carbodiimides according to one or more of Claims 1 to 7 comprising the steps of:
a) partial, preferably < 50%, functionalization of the free NCO groups of aromatic diisocyanates of formula (II) 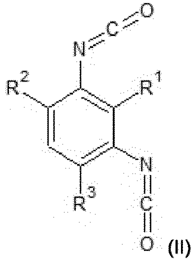 with alcohols of formula NOR^{III} and
b) subsequent carbodiimimidization of the partially functionalized aromatic diisocyanates of formula (II) obtained in step a) to eliminate carbon dioxide at temperatures of 80°C to 200°C in the presence of catalysts and optionally solvent,
wherein R¹ to R³ and R^{III} are as defined above for the compounds of formula (I)

10. Process for producing carbodiimides according to one or more of Claims 1 to 7 comprising carbodiimidization of aromatic diisocyanates of formula (II) 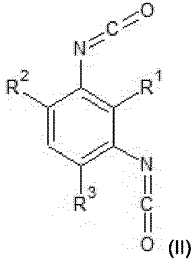 to eliminate carbon dioxide at temperatures of 80°C to 200°C in the presence of catalysts and optionally solvent, wherein before, during or after the carbodiimidization of the diisocyanates monoisocyanates of formula OCN-R^{I} are added and wherein R¹ to R³ and R^{I} are as defined above for the compounds of formula (I).

11. Process according to any of Claims 8 to 10, wherein the aromatic diisocyanates of formula (II) employed are compounds of formulae (III) 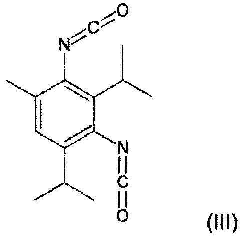 and/or (IV).

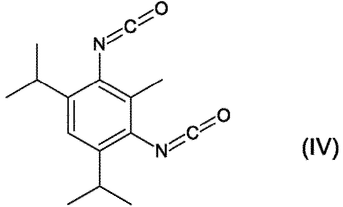

12. Process according to one or more of Claims 8 to 11, wherein R = -NCN-R^{I}, wherein R^{I} is as defined above for the compounds of formula (I) and the melt of the carbodiimide obtained in the carbodiimization is pelletized in unpurified form or after purification.

13. Use of the carbodiimides according to any of Claims 1 to 7 as an inhibitor against hydrolytic decomposition in ester-based polymers, preferably polymers selected from polyester polyols, polyethylene terephthalate (PET), polybutylene terephthalate (PBT), polytrimethylene terephthalate (PTT), copolyesters such as modified polyesters of cyclohexanediol and terephthalic acid (PCTA), thermoplastic polyester elastomers (TPE E), ethylene vinyl acetate (EVA), polylactic acid (PLA) and/or PLA derivatives, polybutylene adipate-terephthalates (PBAT), polybutylene succinates (PBS), polyhydroxyalkanoates (PHA), polyurethane elastomers, preferably thermoplastic polyurethanes (TPU), and blends, preferably PA/PET or PHA/PLA blends.

14. Use of the carbodiimides according to any of Claims 1 to 8 as protection against hydrolytic degradation in thermoplastic polyurethane (TPU).

15. Composition containing at least one inventive carbodiimide according to any of Claims 1 to 7 and at least one ester-based polymer, preferably an ester-based polymer selected from polyester polyols, polyethylene terephthalate (PET), polybutylene terephthalate (PET), polytrimethylene terephthalate (PTT), copolyesters such as modified polyesters of cyclohexanediol and terephthalic acid (PCTA), thermoplastic polyester elastomers (TPE E), ethylene vinyl acetate (EVA), polylactic acid (PLA) and/or PLA derivatives, polybutylene adipate-terephthalates (PBAT), polybutylene succinates (PBS), polyhydroxyalkanoates (PHA), polyurethane elastomers, in particular thermoplastic polyurethanes (TPU), and blends, such as in particular PA/PET or PHA/PLA blends.

## Revendications

1. Carbodiimides de formule (I) 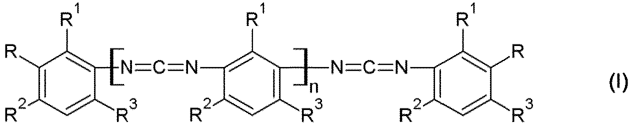 dans laquelle
R peut être identique ou différent et est choisi parmi -NCN-R^{I} et -NHCOOR^{III}, où
R^{I} représente un alkyle en C₁-C₂₂, un cycloalkyle en C₆-C₁₂, un aryle en C₆-C₁₈ ou un aralkyle en C₆-C₁₈, de préférence un triisopropylphényle et
R^{III} représente un radical polyoxyalkylène alkylé,
R¹, R² et R³ représentent chacun indépendamment un groupe méthyle, i-propyle ou n-propyle, l'un des radicaux R¹, R² et R³ sur chaque noyau benzène étant un groupe méthyle et n étant de 0 à 500, de préférence de 1 à 50.

2. Carbodiimides selon la revendication 1, où R¹, R² et R³ représentent chacun indépendamment les uns des autres un groupe méthyle ou i-propyle.

3. Carbodiimides selon la revendication 1 ou 2, où la teneur en carbodiimide est de 2 à 17 % en poids.

4. Carbodiimides selon une ou plusieurs des revendications 1 à 3, où, dans la formule (I), R représente NCN-R^{I}, n est de 0 à 500, de préférence de 1 à 100, de manière particulièrement préférée de 1 à 50, et la teneur en carbodiimide est de préférence de 10 à 17 % en poids, de manière particulièrement préférée de 11 à 15 % en poids, et de manière la plus particulièrement préférée de 13 à 14 % en poids.

5. Carbodiimides selon une ou plusieurs des revendications 1 à 3, où, dans la formule (I), R représente -NHCOOR^{III}, n est de 0 à 20, de préférence de 1 à 10, de manière particulièrement préférée de 3 à 8, et la teneur en carbodiimide est de préférence de 4 à 13 % en poids, de manière particulièrement préférée de 10 à 13 % en poids.

6. Carbodiimides selon une ou plusieurs des revendications 1 à 3, où, dans la formule (I), R représente -NHCOOR^{III} et R^{III} représente un radical polyoxyalkylène alkylé, n est de 0 à 20, de préférence n est de 1 à 10, de manière particulièrement préférée de 1 à 4 et de manière la plus particulièrement préférée de 2 à 3, et la teneur en carbodiimide est de préférence de 2 à 10 % en poids, de manière particulièrement préférée de 4 à 8 % en poids et de manière la plus particulièrement préférée de 5 à 7 % en poids.

7. Carbodiimides selon la revendication 6, où R^{III} représente des éthers de polyéthylèneglycol monoalkylés, de préférence des éthers de monométhyle de polyéthylèneglycol, de préférence avec des masses molaires de 200 à 600 g/mol, de manière particulièrement préférée avec des masses molaires de 350 à 550 g/mol.

8. Procédé de préparation de carbodiimides selon une ou plusieurs des revendications 1 à 7, comprenant les étapes suivantes :
a) la carbodiimidisation de diisocyanates aromatiques de formule (II) 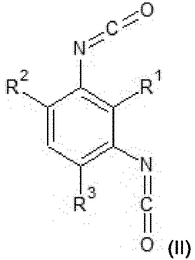 avec clivage de dioxyde de carbone à des températures de 80 °C à 200 °C, en présence de catalyseurs et éventuellement de solvants, et
b) la fonctionnalisation des groupes NCO libres des carbodiimides obtenus dans l'étape a) avec des alcools de formule NOR^{III}, où R¹ à R³ et R^{III} ont les significations indiquées pour les composés de formule (I).

9. Procédé de préparation de carbodiimides selon une ou plusieurs des revendications 1 à 7, comprenant les étapes suivantes :
a) la fonctionnalisation partielle, de préférence < 50 %, des groupes NCO libres de diisocyanates aromatiques de formule (II) 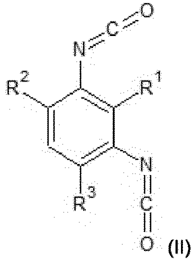 avec des alcools de formule NOR^{III} et
b) la carbodiimidisation subséquente des diisocyanates aromatiques partiellement fonctionnalisés de formule (II) obtenus dans l'étape a) avec clivage de dioxyde de carbone à des températures de 80 °C à 200 °C en présence de catalyseurs et éventuellement de solvants,
où R¹ à R³ et R^{III} ont les significations indiquées pour les composés de formule (I)

10. Procédé de préparation de carbodiimides selon une ou plusieurs des revendications 1 à 7, comprenant la carbodiimidisation de diisocyanates aromatiques de formule (II) 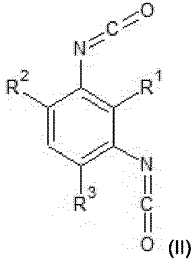 avec clivage de dioxyde de carbone à des températures de 80 °C à 200 °C en présence de catalyseurs et éventuellement de solvants, des monoisocyanates de formule OCN-R^{I} étant ajoutés avant, pendant ou après la carbodiimidisation des diisocyanates, et R¹ à R³ et R^{I} ayant les significations indiquées pour les composés de formule (I).

11. Procédé selon l'une quelconque des revendications 8 à 10, où des composés de formules (III) 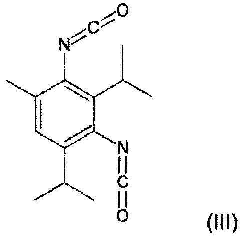 et/ou (IV) 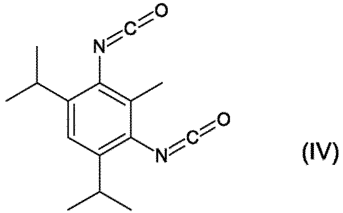 sont utilisés en tant que diisocyanates aromatiques de formule (II).

12. Procédé selon une ou plusieurs des revendications 8 à 11, où R = -NCN-R^{I}, où R^{I} a la signification indiquée pour les composés de formule (I) et la masse fondue du carbodiimide obtenue lors de la carbodiimidisation est non purifiée ou pastillée après purification.

13. Utilisation des carbodiimides selon l'une quelconque des revendications 1 à 7 en tant que protection contre la dégradation hydrolytique dans des polymères à base d'ester, de préférence des polymères choisis parmi les polyesters polyols, le polyéthylène téréphtalate (PET), le polybutylène téréphtalate (PBT), le polytriméthylène téréphtalate (PTT), les copolyesters tels que les polyesters modifiés de cyclohexanediol et d'acide téréphtalique (PCTA), les élastomères polyesters thermoplastiques (TPE E), l'éthylène-acétate de vinyle (EVA), l'acide polylactique (PLA) et/ou les dérivés de PLA, les polybutylène adipate téréphtalates (PBAT), les succinates de polybutylène (PBS), les polyhydroxyalcanoates (PHA), les élastomères de polyuréthane, de préférence les polyuréthanes thermoplastiques (TPU), et les mélanges, tels que de préférence les mélanges PA/PET ou PHA/PLA.

14. Utilisation des carbodiimides selon l'une quelconque des revendications 1 à 8 en tant que protection contre la dégradation hydrolytique dans le polyuréthane thermoplastique (TPU).

15. Composition contenant au moins un carbodiimide selon l'invention selon l'une quelconque des revendications 1 à 7 et au moins un polymère à base d'ester, de préférence un polymère à base d'ester choisi parmi les polyesters polyols, le polyéthylène téréphtalate (PET), le polybutylène téréphtalate (PBT), le polytriméthylène téréphtalate (PTT), les copolyesters tels que les polyesters modifiés de cyclohexanediol et d'acide téréphtalique (PCTA), les élastomères polyesters thermoplastiques (TPE E), l'éthylène-acétate de vinyle (EVA), l'acide polylactique (PLA) et/ou les dérivés de PLA, les polybutylène adipate téréphtalates (PBAT), les succinates de polybutylène (PBS), les polyhydroxyalcanoates (PHA), les élastomères de polyuréthane, notamment les polyuréthanes thermoplastiques (TPU), et les mélanges, tels que notamment les mélanges PA/PET ou PHA/PLA.
